# EUROPEAN PATENT SPECIFICATION

(11) **EP 3 873 235 B1**
(45) Date of publication and mention of the grant of the patent: **19.02.2025**
(21) Application number: 19805742.4
(22) Date of filing: 31.10.2019
(51) Int. Cl.: A23L 33/105, A61K 31/198, A61K 36/84, A61K 36/88

(54) **PHYTO-THERAPYC COMPOSITIONS USEFUL TO IMPROVE THE QUALITY OF SLEEP AND IN THE TREATMENT OF INSONNIA, ANXIETY AND DEPRESSION**
PHYTOTHERAPIEZUSAMMENSETZUNGEN ZUR VERBESSERUNG DER SCHLAFQUALITÄT UND ZUR BEHANDLUNG VON SCHLAFSTÖRUNGEN, ANGST UND DEPRESSION
COMPOSITIONS PHYTOTHÉRAPEUTIQUES UTILES POUR AMÉLIORER LA QUALITÉ DU SOMMEIL ET DANS LE TRAITEMENT DE L'INSOMNIE, DE L'ANXIÉTÉ ET DE LA DÉPRESSION

(30) Priority: 02.11.2018 IT 201800010002
(43) Date of publication of application: 08.09.2021
(73) Proprietor: Giovannone, Daniele, 03100 Arpino - Frosinone (IT)
(72) Inventor: Giovannone, Daniele, 03100 Arpino - Frosinone (IT)
(74) Representative: Di Bernardo, Antonio
(86) International application number: PCT/IB2019/059354
(87) International publication number: WO 2020/089831

(56) References cited:
- CN-A- 104 758 490
- US-A1- 2006 246 129
- ADRIAN L. LOPRESTI ET AL: "Saffron ( Crocus sativus ) for depression: a systematic review of clinical studies and examination of underlying antidepressant mechanisms of action : SAFFRON ( Crocus sativus ) FOR DEPRESSION", HUMAN PSYCHOPHARMACOLOGY. CLINICAL AND EXPERIMENTAL., vol. 29, no. 6, 1 November 2014 (2014-11-01), XX, pages 517 - 527, XP055524333, ISSN: 0885-6222, DOI: 10.1002/hup.2434
- HEATHER ANN HAUSENBLAS ET AL: "Saffron (Crocus sativus L.) and major depressive disorder: a meta-analysis of randomized clinical trials", JOURNAL OF INTEGRATIVE MEDICINE, vol. 11, no. 6, 1 November 2013 (2013-11-01), pages 377 - 383, XP055626282, ISSN: 2095-4964, DOI: 10.3736/jintegrmed2013056
- ANDREATINI R ET AL: "Effect of valepotriates (Valerian extract) in generalized anxiety disorder: a randomized placebo-controlled pilot study", MEDICINAL & AROMATIC PLANTS ABSTRACTS, SCIENTIFIC PUBLISHERS, SCIENTIFIC PUBLISHERS, NEW DELHI - INDIA, vol. 25, no. 5, 1 October 2003 (2003-10-01), XP018015414, ISSN: 0250-4367
- ABASCAL K ET AL: "Nervine herbs for treating anxiety", ALTERNATIVE AND COMPLEMENTARY THERAPIES, MARY ANN LIEBERT, LARCHMONT, NY, US, vol. 10, no. 6, 1 December 2004 (2004-12-01), pages 309 - 315, XP009110219, ISSN: 1076-2809, DOI: 10.1089/ACT.2004.10.309
- KIMURA K ET AL: "l-Theanine reduces psychological and physiological stress responses", BIOLOGICAL PSYCHOLOGY, NORTH-HOLLAND PUB., AMSTERDAM, NL, vol. 74, no. 1, 1 January 2007 (2007-01-01), pages 39 - 45, XP027017468, ISSN: 0301-0511, [retrieved on 20061122]

## Description

### Technical field of invention

The present invention describes the preparation of a composition comprising phytotherapeutic preparations of valerian, L-theanine and saffron, each one characterized by a sufficient presence of the specific active ingredients and mixed together in an adequate proportion to give a high biological response. In particular, this composition is effective for oral administration in order to improve mood and sleep quality in anxious people, which are subject to panic attacks or depressed, and in order to reduce conditions of anxiety, panic and depression.

### Background art

Depression is a known pathological condition that can take mild forms, for example characterized by feelings of sadness, tiredness or inadequacy, up to reach thoughts of death and suicide in the most serious cases. It may be present for a transitional period, such as postpartum depression, but it may also persist indefinitely. It can also have serious interference with people's daily lives and can lead to deviant behaviour such as alcoholism and drug use. Depression is often accompanied by anxiety and sleep disorders.

Anxiety is an equally well-known psychological condition, which is generally manifested with restlessness and nervousness, even when not justified by objective causes; in milder cases it can be considered as a characteristic of the character rather than a pathology. Even in mild cases it interferes with everyday life, for example with the personal performances when taking examinations in school activities, in completing tasks in working life, in public speaking and in other situations; it is often accompanied by sleep disturbances, particularly in difficulty falling asleep, and possible mood disorders. In the most serious cases it can lead to panic attacks, moments of uncontrolled terror that cause a great momentary disturbance to the person and that make him even more insecure in the future, also hindering social relations.

Sleep disorders manifest themselves as a difficulty in falling asleep, in maintaining a long sleep condition, with nocturnal awakenings, with early morning awakenings, and lead to a decrease in the quantity and quality of rest. Consequently, they influence mood, personal performance and social relations. Sleep disorders are often present in anxious or depressed subjects, but can also occur in subjects who are not anxious nor depressed, either occasionally, such as changing the time zone after an air travel, or continuously, for example in people who take shifts of night work.

For the therapy of anxiety conditions and for the treatment of depression and panic attacks, numerous drugs exist, belonging to different chemical classes, based on different mechanisms of action and with various efficacy: serotonin reuptake inhibitors are widespread, the foremost of which is fluoxetine. However, there is a wide selection of active ingredients, so the doctor can customize the treatment by choosing the most suitable drug for the patient and graduating the dosage.

The treatment of sleep disorders is a complicated field (for example, it is not yet clear what sleep is for), which must be addressed by specialist doctors in the most complex cases. In many people, however, situations of mild or moderate sleep disorders occur, which in any case worsen the quality of rest: for example, it may be difficult to fall asleep, there may be sudden awakenings during sleep, difficulty in reaching the deep sleep phase. In many cases it is not possible to identify a precise organic cause, or a psychological cause is known: in particular, stress and anxiety condition can negatively affect the quality and duration of sleep. There are numerous drugs useful for promoting the onset and maintenance of sleep, among which benzodiazepines are particularly successful; other drugs such as barbiturates are useful only in the most severe cases.

All these antidepressant, anxiolytic and hypnotic drugs have high efficacy but also important side effects, which can cause the patient to not respect the indicated dosage or to interrupt the treatment. Furthermore, some patients, particularly those with mild or medium-sized problems, have a strong aversion to use medications and to seek medical attention: many people prefer to go to the pharmacist or herbalist, looking for a solution perceived like a less pharmaceutical and more natural one.

The use of substances of plant origin (or, more rarely, animal) as a remedy for various pathological conditions has always been known and it is described as a herbalist practice or phytotherapy: human experience has progressively selected the most effective herbs and most suitable preparation methods. The most used preparations are drugs, powders, dry and fluid extracts, tinctures etc., each obtained following reliable techniques described in the Pharmacopoeias. Among the numerous herbal products traditionally used as mild hypnotics, typically in infusion, are e.g. tea (*Camelia sinensis,* leaf) chamomile *(Chamomile matricaria,* flower), poppy *(Papaverum somnifero)* linden tree (leaf), lemon balm, hawthorn etc.; you can also find ready-to-use sachets, also mixed together or with other herbs (e.g. fennel or other digestive herbs). These infusions have a proven, but really modest and very subjective, efficacy.

In the modern conception of phytotherapy, the active ingredients contained in the various preparations are separated, identified and measured, then evaluating their effectiveness; in fact it is widely known that the presence of active ingredients in medicinal herbs is widely variable based on numerous and often uncontrollable factors such as climate, soil, harvest time and others. The use of titrated and standardized preparations allows to obtain a good reproducibility in the composition and efficacy of phytotherapeutic formulations: the most elaborate and most modern products use known active vegetable ingredients which are exactly dosed and presented in pharmaceutical forms, for example capsules or tablets. Also, for the treatment of anxiety and depression specific formulations are available, containing the active ingredients already listed, but in exactly defined quantities. An example is Valmane^{®}, which contains as a single active ingredient a dry extract of valerian root, recommended dose 125 mg/ day. In addition to those already listed, a particular emphasis is given to hypericum, a plant whose antidepressant effect has been known for centuries, but which also has important side effects; in this case the use of formulations with a known potency and reproducible dosage is of particular importance.

Often herbal formulations are added with melatonin, a hormone involved in regulating the sleep-wake cycle, the introduction of which is relatively recent. Although generally obtained by chemical synthesis, it is perceived by patients as a natural product and is considered particularly effective; there are also formulations containing only melatonin as active ingredient.

Nevertheless, it is not always possible to identify all the active compounds present in a herbal preparation and to attribute each one a biological effect: the simultaneous presence in the same preparation of many substances (often chemically similar to each other) involves the possible interaction between the substances themselves, for example one can favour (or inhibit) the absorption of the other, and thus vary the effectiveness of the preparation itself. In this case we speak of synergy. Compositions comprising several active ingredients are commercially available, for example, under the name of Sominex (including passionflower, valerian and cretaegus) Songha night (valerian extract 120 mg, melissa extract 80 mg) Neurapas (hypericum e.g. 240 mg, passionflower e.g. 128 mg, valerian e.g. 112 mg).

The journal article by Adrian L. Lopresti et al. (Human Psychopharmacology. Clinical and Experimental vol. 29, n. 6 (2014), pages 517-527) provides a systemic review of the completed clinical studies regarding saffron and depression; in particular, six previous clinical studies were analysed.

In the journal article by Heather Ann Hausenblas et al. (J. of integrative medicine, vol. 11, n. 6 (2013), pages 377-383) a meta-analysis of published, randomized controlled trials that examine the effects of saffron supplementation on the symptoms of depression in subjects with depressive disorders was performed.

In Andreatini R. et al. (Medicinal & Aromatic Plants Abstracts, Scientific Publishers, New Dheli - India, vol. 25, n. 5 (2003)) and in Abascal K. et al. (Alternative and Complementary Therapies vol. 10, n. 6 (2004) pages 309-315) the use of extracts of various nervine herbs, including valerian, to treat various conditions such as anxiety and panic was described.

In Kimura K. *et al.* a study was performed to assess the influence of L-theanine on stress and anxiety (Kimura K. et al., Biological Psychology vol. 74, n. 1 (2007), pages 39-45).

United States patent application US2006246129 relates to a composition comprising, among others, an extract of valerian and L-theatine for the treatment of sleep disorders.

Lastly, Chinese patent application CN104758490 describes a traditional Chinese medicine for treating tinnitus caused by stress or depression, which includes, among other extracts, also that of saffron and valerian.

### Summary of invention

A surprising synergistic effect has now been found between some of the active plant extracts traditionally used in phytotherapy: a composition comprising valerian extract, saffron and theanine has shown considerable efficacy in the treatment of anxiety and depressive conditions, and a consequent improvement in quality of sleep. The effectiveness is further increased when phytic acid, a component normally present in many vegetables but of which no phytotherapeutic efficacy has been known until now, is added to the mixture of the three phytotherapeutic products.

### Detailed description of invention

The invention consists of a mixture of at least three plant extracts, in proportions varying within defined limits and preferably analysed for their content in active ingredients, for use in treating anxiety. Said mixture comprises:
a) a preparation of valerian (*Valeriana officinalis),* preferably in the form of a dry extract with a known titration in valerenic acids,
b) saffron *(Crocus sativus L.)* preferably in the form of a dry powder with a known titration in safranal or in crocin
c) de-caffeinated tea (*Camelia sinensis),* a preparation with reduced content of theine in the form of a dried leaf or dry powder or extract, preferably with a known titration in L-theanine.

In a particular embodiment, in place of the leaf tea or an extract thereof, the composition may comprise L-theanine in the form of a pure active ingredient. In one embodiment, the composition is formulated in capsules or tablets or other pharmaceutical form suitable to ensure an oral administration at the exact dose of the active ingredients. In a particular embodiment, the composition of the invention is formulated in gastro-resistant coated tablets. In another embodiment, the composition is dissolved in water or in a non-toxic solvent such as e.g. ethanol or glycerine, optionally mixed with water, for example in an aqueous mixture containing more than 50% of ethanol or glycerine.

The above composition can be added with excipients such as lubricants, fillers, flavouring and sweetening agents, disintegrating and other useful components depending on the preparation technology.

It has in fact been noted that a balanced composition of the three ingredients mixed together is particularly effective in the treatment of anxiety conditions (anxiety, panic attacks), depression and in promoting a natural and relaxing sleep. As illustrated in the following examples, the mixture of the three components has a better effect in terms of potency (efficacy in relation to the dose) and reproducibility (number of subjects with a positive response) compared to the use of the single components and compared to other mixtures of active ingredients known as relaxing or hypnotic.

The mixture can comprise the three products both in the form of drugs, in particular as a dry powder, both in the form of extracts, such as dry extract or fluid extract; in a particular embodiment, the invention consists in mixing extracts or drugs having defined titration. Hence, the extracts are not dosed on the base of extraction ratio, as the traditional use, but on the basis of the titration of the active ingredient: this processing practice entails additional costs for the analysis but it allows to obtain a good standardization of the end product and therefore a good reproducibility in its effectiveness.

For example, we refer to the content in valerenic acids, family of compounds characterizing valerian and its extracts, in order to relate the quantity of raw material to be used in the composition. For tea-based preparations, instead of theine, we refer to the content of L-theanine, a little-known amino acid that we believe is responsible for the relaxing effect; in the traditional drink, this effect is contrasted by the presence of theine (caffeine), a known stimulant. To dose saffron preparations, we refer to the sum of the major active ingredients crocin, safranal and picrocrocin; this value is considered representative of the concentration and quality of the saffron preparation.

In a particular embodiment, the invention consists of the mixture of finely divided powders and formulated in granulates, capsules or tablets, for use in treating anxiety:
- the granulates are particularly indicated when a localized absorption in the mouth mucosa is desired, without involving the stomach and the intestine
- effervescent granules or effervescent tablets are preferred for the administration of the active ingredients in suspension or solution, the powders are then previously diluted in water
- the capsules and tablets are used when absorption into the intestine is desired, or to mask the taste of the preparation
- coated tablets are used when it is desired to protect the active ingredients from stomach acidity, when a delayed or prolonged release is desired, or to mask the taste of the preparation.

In all these cases, excipients are used to adapt the characteristics of the mixture of active ingredients to the specific preparation technology of the formulation.

In a further embodiment, the composition for use of invention is formulated as a solution or suspension of the active ingredients in water, ethyl alcohol, glycerine or a mixture of said solvents. This formulation allows a more accurate and customizable administration, dosing the number of drops to be taken; the drops can be either diluted in water or taken without dilution, in particular when a localized absorption in the oral mucosa is desired.

In further embodiments, the composition for use of the invention is added with other active ingredients useful for promoting sleep and/or in the therapy of anxiety and depression, such as e.g. melatonin, 5-hydroxytryptophan, extracts of chamomile, hypericum, linden, hawthorn, poppy or other drugs, or with similar plant extracts.

In a further embodiment, the composition for use of the invention is added with other useful active ingredients, such as for example vitamins, in particular the B vitamins, more particularly vitamins from the folate group.

In a particular embodiment, to the mixture of the three active components is added a source of phytic acid (more correctly called inositol hexaphosphate, or myo-inositol hexaphosphate, or IP6) a component present in numerous plant sources, including those of food or phytotherapy interest. It can be found in all plant cells, mainly as salt (calcium, magnesium or iron). The actual phytic acid is often accompanied by other similar products, for example inositol penta-phosphate, tetra-phosphate and the like: therefore, we speak of phytates as a family of products.

The phytic acid content presents in drugs and plant foods can greatly vary, both on the basis of agronomic parameters (type and variety of the plant, growth soil, harvest time, etc.) both on depending of the subsequent processing: for example the steps of harvesting, drying, maceration in water or solvents, treatments with acids, fermentation can lead to a drastic reduction in the content of phytates, due to activation of phytases or to simple thermo-acid degradation. In the composition of the invention, preparations are used, for example alimentary flours particularly rich in phytic acid (soya, almond, rice, wheat flour), or salts of inositol hexaphosphate, for example the mono-, nona-, or dodeca-sodium salts, or even a titrated aqueous solution; in all cases we refer to the content of myo-inositol hexaphosphate (IP6).

To date, no phytotherapeutic or nutritional activities of phytic acid and its salts are known, the same is not described in the pharmacopoeia nor is it considered a product of herbal or phytotherapeutic interest; in particular not as active on the nervous system.

It has now surprisingly been discovered that the presence of phytic acid (or its salts), added to the mixture of valerian, tea and saffron described above, increases the efficacy of the product. It is therefore possible to obtain a biological effect with reduced doses of herbal extracts, as described in the following examples.

### Example 1

A mixture is prepared with the following composition:

| | |
|---|---|
| valerian powder | 600 g |
| saffron powder | 5 g |
| decaffeinated tea powder | 1500 g |

For the valerian a root powder titrated to 100 ppm in valerenic acids is used, for the saffron a powder of stigmas titrated at 200 ppm in active ingredients is used, for tea a de-caffeinated preparation in a soluble powder with L-theanine content 0.2% is used. The powders are carefully dry mixed and then subjected to grinding and screening on a sieve with 500 micron wire mesh, then stored in an opaque plastic jar. The mixture is divided into doses for administration.

### Example 2

A mixture is prepared with the following composition:

| | |
|---|---|
| valerian dry extract | 60 g |
| saffron powder | 5 g |
| decaffeinated tea powder | 1500 g |

For valerian, a dry extract titrated to 0.8% in valerenic acids is used, for saffron a preparation titrated in active ingredients of 3.5%, on the market under the name of affron^{®}, is used, for tea a decaffeinated soluble powder with a total content of L-theanine of 20 g is used.

The powders are carefully mixed, ground and screened as described in Example 1, then stored in an opaque plastic jar.

### Example 3

A mixture is prepared with the following composition:

| | |
|---|---|
| valerian dry extract | 60 g |
| saffron powder | 5 g |
| pure L-theanine | 104 g |

For valerian a dry extract titrated to 0.8% in valerenic acids is used, for saffron a preparation titrated to 3.5% commercialised under the name of affron^{®} is used. In place of tea, pure theanine in powder form with a titre> 95% is used.

The powders are carefully mixed, ground and screened as described above, then stored in an opaque plastic jar.

### Example 4

A mixture is prepared with the following composition:

| | |
|---|---|
| valerian dry extract | 60 g |
| saffron powder | 5 g |
| pure L-theanine | 104 g |
| sodium inositol hexaphosphate | 100 g |

For valerian a dry extract titrated to 0.8% in valerenic acids is used, for saffron a preparation titrated to 3.5% is used. Commercial sodium inositol hexaphosphate and theanine are added in the showed quantities. The powders are carefully mixed, ground and screened as described above, then stored in an opaque plastic jar.

### Example 5

A mixture is prepared with the following composition:

| | |
|---|---|
| valerian dry extract | 90 g |
| saffron powder | 5 g |
| pure L-theanine | 100 g |
| almond flour | 600 g |

For valerian, saffron and theanine the preparations listed in the previous example are used; as source of inositol hexaphosphate, almond flour with a content of 8% in IP6 is used.

The powders are carefully mixed, ground and screened as described above, then stored in an opaque plastic jar.

### Example 6

To 100 g of the powder mixture of Example 3, 235 mg of (6R)5-methyltetrahydrofolic acid glucosamine salt, commercially under the name Quatrefolic^{®}, are added, then well mixed.

### Example 7

To 100 g of the powder mixture of Example 4, 150 mg of (6R)5-methyltetrahydrofolic acid glucosamine salt, commercially under the name Quatrefolic^{®}, are added, then well mixed.

### Example 8

To 100 g of the powder mixture of Example 4,185 mg of pure melatonin are added, then well mixed.

### Example 9

To 100 g of the powder mixture of Example 4, 100 g of yeast enriched in S-adenosylmethionine are added, then well mixed.

### Example 10

To 100 g of the powder mixture of Example 3, 200 g of S-adenosylmethionine inositol phosphate salt, in the market as SAMphyt^{®}, are added; then well mixed.

### Example 11

A mixture is prepared with the following composition:

| | |
|---|---|
| valerian dry extract | 60 g |
| saffron powder | 5 g |
| L-theanine | 104 g |
| phytic acid water solution | 300 ml |
| purified water | q.b. to 1000 |

The powdered components are added to about 700 ml of purified water, swirling until a yellow solution is obtained, then a 50% aqueous solution of phytic acid is added as a source of inositol hexaphosphate. It is mixed well and bring to volume. It is divided into dark glass containers fitted with a dropper cap.

### Example 12

The formulation described in Example 3 is tested in simulated gastrointestinal absorption tests, in vitro, using a Caco-2 type intestinal epithelium model in well cells. The model allows to test an aqueous solution of active ingredients and to evaluate their distribution on both sides of a membrane made up of Caco-2 cells distributed in a continuous monolayer: the solution being analysed is placed on the apical side, corresponding in vivo to the intestinal lumen. The active ingredients can migrate to the basolateral side of the membrane, corresponding *in vivo* to the intestinal tissue supplied with blood. The concentration of the active ingredients on both sides of the membrane is evaluated at time intervals by HPLC analysis. A sample of the initial solution is stored outside the test well, under the same pH and temperature conditions, to evaluate the possible spontaneous degradation of the active ingredient (reference) in the same time intervals. At the end of the experiment the integrity of the membrane is verified by microscopic observation; the experiment is repeated if the membrane is damaged.

The results obtained are shown in Table 1 below. The concentration of active ingredient in the basolateral compartment of the well is reported, expressed as a percentage of the initial concentration administered on the apical side. The complete permeability of the membrane involves an equal distribution of the PA in the two sides, corresponding to a value of 50% in the basolateral side. No damage to the membrane was found in any experiment.

| | | | | | |
|---|---|---|---|---|---|
| Time (min) | 0 | 15 | 30 | 60 | 120 |
| safranal | 0 | 14 | 28 | 36 | 45 |
| valerenic acids (sum) | 0 | 5 | 11 | 20 | 28 |
| L-theanine | 0 | 24 | 36 | 45 | 50 |

Table 1: simulated intestinal absorption of active ingredients of the formulation 3. As evidenced by the data in the table, *in vitro* the formulations described allow a passage of all the active ingredients from the administration (apical) side to the basolateral side, crossing the intact intestinal membrane; this corresponds to an absorption of the active ingredients in vivo in an enteric administration (capsule, soft capsule, tablet, gastroresistant tablet or the like).

### Example 13

The test described in the previous example is repeated using the formulation of Example 4; the data shown in Table 2 are obtained.

| | | | | | |
|---|---|---|---|---|---|
| Time (min) | 0 | 15 | 30 | 60 | 120 |
| safranal | 0 | 22 | 35 | 40 | 48 |
| valerenic acids (sum) | 0 | 14 | 24 | 38 | 40 |
| L-theanine | 0 | 32 | 44 | 50 | 50 |

Table 2: simulated intestinal absorption of active ingredients of the formulation 4. The presence of phytic acid (inositol hexaphosphate, IP6) as a monosodium salt further increases the absorption of the active ingredients, without thereby damaging the intestinal membrane: as can be seen by comparing the data in Table 2 with those in Table 1, absorption it is faster and more complete for all the active ingredients administered.

### Example 14

The formulation described in Example 6 is tested in simulated oral absorption tests, *in vitro,* using a model of epithelium of the reconstituted oral mucosa HOE (Human Oral Epithelium) of SkinEthic, in well cells. One operates as described in Example 12, evaluating the concentration of the active ingredients in the apical and basolateral compartments at time intervals. For the control of spontaneous degradation and membrane integrity one operates as described in example 12. The results obtained are shown in the table below. No damage to the membrane was found in any experiment.

| | | | | |
|---|---|---|---|---|
| Time (min) | 0 | 5 | 10 | 20 |
| safranal | 0 | 25 | 35 | 42 |
| valerenic acids (sum) | 0 | 22 | 30 | 40 |
| L-theanine | 0 | 28 | 42 | 50 |

Table 3: simulated intestinal absorption of active ingredients of the formulation 6. As evidenced by the data in the table, *in vitro* the formulation described give a rapid passage of all active ingredients from the administration (apical) side to the basolateral side, crossing the intact HOE membrane; this corresponds to an absorption of the active ingredients *in vivo* in a local release administration (stick pack, soluble granulate, sublingual tablet, solution or tincture, candy, chewable gum and the like).

### Example 15

The formulation described in Example 11 is tested in simulated oral absorption tests as described in Example 14; the following results are obtained:

| | | | | |
|---|---|---|---|---|
| Time (min) | 0 | 5 | 10 | 20 |
| safranal | 0 | 32 | 44 | 50 |
| valerenic acids (sum) | 0 | 28 | 33 | 42 |
| L-theanine | 0 | 39 | 48 | 50 |

Table 4: simulated intestinal absorption of active ingredients of the formulation 11. The presence of myo-inositol hexaphosphate (as sodium salt) further increases the absorption of the active ingredients, without damaging the oral membrane: as can be seen by comparing the data in Table 4 with those in Table 3, in the presence of phytic acid the absorption is faster and more complete for all active ingredients administered.

## Claims

1. A food, pharmaceutical or nutraceutical composition for use in treating anxiety comprising
a) saffron, and
b) *valeriana officinalis,* and
c) at least one source of L-theanine.

2. The composition for use of claim 1 wherein the L-theanine source has a low content of caffeine and theobromine.

3. The composition for use of claims 2 wherein the L-theanine source is a powder of pure L-theanine.

4. The composition for use of claims 1-3 wherein valeriana is in form of an extract, preferably a dry extract, more preferably dry extract having a known titration in valerenic acids.

5. The composition for use of claims 1-4 wherein saffron is in form of a powder, preferably having a known titration in active ingredients, preferably with a known titration in crocin, safranal and picrocrocin.

6. A composition comprising any one of the composition of claims 1-5 for use in treating anxiety, wherein a source of inositol hexaphosphate is added, preferably as a watersoluble salt thereof, more preferably as a sodium salt.

7. The composition for use of claim 6, **characterized in that** said source of inositol hexaphosphate is phytic acid, preferably in water solution.

8. The composition for use of claim 6, **characterized in that** said source of inositol hexaphosphate is almond flour, preferably with a known titration in inositol hexaphosphate.

9. A pharmaceutical or nutraceutical formulation for oral use comprising a composition according to any one of the claims from 1 to 8 for use in treating anxiety, said composition being in powder form, said pharmaceutical or nutraceutical formulation being preferably as stick-pack, capsule, soft capsule, tablet, coated tablet, effervescent tablet, orosoluble tablet, granulate, effervescent granulate.

10. A pharmaceutical or nutraceutical formulation comprising a composition according to any one of the claims from 1 to 8 for use in treating anxiety, for the release in the mouth of one or more active ingredients comprised in the group consisting of L-theanine, crocin, picrocrocin, safranal, valerenic acids, said pharmaceutical od nutraceutical formulation being stick-pack, orosoluble granulate, sublingual tablet, chewable gum, candy.

11. A pharmaceutical or nutraceutical formulation comprising a solution of one of the compositions according to any of claims from 1 to 8 for use in treating anxiety in a pharmaceutically acceptable solvent, preferably a solvent comprising water, more preferably water.

12. The pharmaceutical or nutraceutical formulation according to any one of claims from 9 to 11 for use in treating anxiety, titrated for the crocin, picrocrocin and safranal, valerenic acids and in L-theanine content, preferably free of caffeine and theobromine.

## Patentansprüche

1. Lebensmittel-, pharmazeutische oder nutrazeutische Zusammensetzung zur Verwendung bei der Behandlung von Angstzuständen, umfassend
a) Safran und
b) *Valeriana officinalis,* und
c) mindestens eine L-Theanin-Quelle.

2. Zusammensetzung zur Verwendung nach Anspruch 1, wobei die L-Theanin-Quelle einen geringen Gehalt an Koffein und Theobromin aufweist.

3. Zusammensetzung zur Verwendung nach Anspruch 2, wobei die L-Theanin-Quelle ein Pulver aus reinem L-Theanin ist.

4. Zusammensetzung zur Verwendung nach Anspruch 1 bis 3, wobei Valeriana in Form eines Extrakts ist, bevorzugt eines Trockenextrakts, bevorzugter eines Trockenextrakts mit einem bekannten Gehalt an Valerensäuren.

5. Zusammensetzung zur Verwendung nach den Ansprüchen 1 bis 4, wobei Safran in Form eines Pulvers ist, bevorzugt mit einem bekannten Gehalt an aktiven Bestandteilen, bevorzugt mit einem bekannten Gehalt an Crocin, Safranal und Picrocrocin.

6. Zusammensetzung, umfassend eine der Zusammensetzungen der Ansprüche 1 bis 5 zur Verwendung bei der Behandlung von Angstzuständen, wobei eine Inositolhexaphosphat-Quelle zugegeben wird, bevorzugt als ein wasserlösliches Salz davon, bevorzugter als ein Natriumsalz.

7. Zusammensetzung zur Verwendung nach Anspruch 6, **dadurch gekennzeichnet, dass** die Inositolhexaphosphat-Quelle Phytinsäure ist, bevorzugt in Wasserlösung.

8. Zusammensetzung zur Verwendung nach Anspruch 6, **dadurch gekennzeichnet, dass** die Inositolhexaphosphat-Quelle Mandelmehl ist, bevorzugt mit einem bekannten Gehalt an Inositolhexaphosphat.

9. Pharmazeutische oder nutrazeutische Formulierung zur oralen Verwendung, umfassend eine Zusammensetzung gemäß einem der Ansprüche 1 bis 8 zur Verwendung bei der Behandlung von Angstzuständen, wobei die Zusammensetzung in Pulverform ist, und die pharmazeutische oder nutrazeutische Formulierung bevorzugt als Stickpack, Kapsel, Weichkapsel, Tablette, beschichtete Tablette, Brausetablette, Schmelztablette, Granulat, Brausegranulat vorliegt.

10. Pharmazeutische oder nutrazeutische Formulierung, umfassend eine Zusammensetzung gemäß einem der Ansprüche 1 bis 8 zur Verwendung bei der Behandlung von Angstzuständen, zur Freisetzung eines oder mehrerer Wirkstoffe im Mund, die in der Gruppe umfasst sind, bestehend aus L-Theanin, Crocin, Picrocrocin, Safranal und Valerensäuren, wobei die pharmazeutische oder nutrazeutische Formulierung ein Stickpack, ein schmelzbares Granulat, eine Sublingualtablette, ein Kaugummi, oder ein Bonbon ist.

11. Pharmazeutische oder nutrazeutische Formulierung, umfassend eine Lösung einer der Zusammensetzungen gemäß einem der Ansprüche 1 bis 8 zur Verwendung bei der Behandlung von Angstzuständen in einem pharmazeutisch akzeptablen Lösungsmittel, bevorzugt einem Lösungsmittel, umfassend Wasser, bevorzugter Wasser.

12. Pharmazeutische oder nutrazeutische Formulierung gemäß einem der Ansprüche 9 bis 11 zur Verwendung bei der Behandlung von Angstzuständen, titriert auf den Gehalt an Crocin, Picrocrocin und Safranal, Valerensäuren und L-Theanin, bevorzugt frei von Koffein und Theobromin.

## Revendications

1. - Composition alimentaire, pharmaceutique ou nutraceutique pour utilisation dans le traitement de l'anxiété, comprenant
a) du safran, et
b) valeriana officinalis, et
c) au moins une source de L-théanine.

2. - Composition pour utilisation selon la revendication 1, dans laquelle la source de L-théanine a une faible teneur en caféine et en théobromine.

3. - Composition pour utilisation selon la revendication 2, dans laquelle la source de L-théanine est une poudre de L-théanine pure.

4. - Composition pour utilisation selon l'un des revendications 1 à 3, dans laquelle la valériane se présente sous la forme d'un extrait, de préférence un extrait sec, de façon davantage préférée un extrait sec ayant un titrage connu en acides valéréniques.

5. - Composition pour utilisation selon l'une des revendications 1 à 4, dans laquelle le safran se présente sous la forme d'une poudre, de préférence ayant un titrage connu en principes actifs, de préférence avec un titrage connu en crocine, safranal et picrocrocine.

6. - Composition comprenant l'une quelconque de la composition selon les revendications 1 à 5 pour utilisation dans le traitement de l'anxiété, dans laquelle une source d'hexaphosphate d'inositol est ajoutée, de préférence sous forme d'un sel hydrosoluble de celui-ci, de façon davantage préférée sous forme d'un sel de sodium.

7. - Composition pour utilisation selon la revendication 6, **caractérisée par le fait que** ladite source d'hexaphosphate d'inositol est l'acide phytique, de préférence en solution aqueuse.

8. - Composition pour utilisation selon la revendication 6, **caractérisée par le fait que** ladite source d'hexaphosphate d'inositol est la farine d'amande, de préférence avec un titrage connu en hexaphosphate d'inositol.

9. - Formulation pharmaceutique ou nutraceutique à usage oral comprenant une composition selon l'une quelconque des revendications 1 à 8 pour utilisation dans le traitement de l'anxiété, ladite composition étant sous forme de poudre, ladite formulation pharmaceutique ou nutraceutique étant de préférence sous forme de sachet tubulaire, de gélule, de gélule molle, de comprimé, de comprimé enrobé, de comprimé effervescent, de comprimé orosoluble, de granulé, de granulé effervescent.

10. - Formulation pharmaceutique ou nutraceutique comprenant une composition selon l'une quelconque des revendications 1 à 8 pour utilisation dans le traitement de l'anxiété, pour la libération dans la bouche d'un ou plusieurs principes actifs compris dans le groupe consistant en la L-théanine, la crocine, la picrocrocine, le safranal, les acides valéréniques, ladite formulation pharmaceutique ou nutraceutique étant un sachet tubulaire, un granulé orosoluble, un comprimé sublingual, une gomme à mâcher, un bonbon.

11. - Formulation pharmaceutique ou nutraceutique comprenant une solution d'une des compositions selon l'une quelconque des revendications 1 à 8 pour utilisation dans le traitement de l'anxiété dans un solvant pharmaceutiquement acceptable, de préférence un solvant comprenant de l'eau, de façon davantage préférée de l'eau.

12. - Formulation pharmaceutique ou nutraceutique selon l'une quelconque des revendications 9 à 11 pour utilisation dans le traitement de l'anxiété, titrée en crocine, picrocrocine et safranal, acides valéréniques et en teneur en L-théanine, de préférence exempte de caféine et de théobromine.
